# EUROPÄISCHE PATENTSCHRIFT

(11) **EP 2 919 893 B1**
(45) Veröffentlichungstag und Bekanntmachung des Hinweises auf die Patenterteilung: **31.05.2017**
(21) Anmeldenummer: 13792316.5
(22) Anmeldetag: 12.11.2013
(51) Int. Cl.: B01D 61/36, C02F 3/28, C02F 11/04, C12M 1/107

(54) **SELEKTIVE ABTRENNUNG VON WASSER MITTELS MEMBRANVERFAHREN IN EINEM ANAEROBEN BIOPROZESS**
SELECTIVE REMOVAL OF WATER BY MEANS OF MEMBRANE PROCESSES IN AN ANAEROBIC BIOPROCESS
SÉPARATION SÉLECTIVE D'EAU AU MOYEN DE PROCÉDÉS À MEMBRANE DANS UN BIOPROCESSUS ANAÉROBIE

(30) Priorität: 13.11.2012 AT 505082012
(43) Veröffentlichungstag der Anmeldung: 23.09.2015
(73) Patentinhaber: Technische Universität Wien, 1040 Wien (AT)
(72) Erfinder: HERWIG, Christoph, A-1130 Wien (AT); SEIFERT, Arne, 1120 Wien (AT)
(74) Vertreter: Gassner, Birgitta
(86) Internationale Anmeldenummer: PCT/EP2013/073587
(87) Internationale Veröffentlichungsnummer: WO 2014/076062

(56) Entgegenhaltungen:
- WO-A1-2006/137808
- WO-A1-2011/095805
- WO-A1-2012/133386
- WO-A2-2006/119052
- US-A1- 2009 117 631

## Beschreibung

Die Erfindung betrifft ein Verfahren zur selektiven Abscheidung von Wasser aus Prozessflüssigkeiten, dadurch gekennzeichnet, dass Wasser bei der Durchführung eines Bioprozesses mittels eines Membranmoduls selektiv abgeschieden wird.

Die Entwicklung und Verbesserung von CO₂-neutralen Energieerzeugungsprozessen ist ein zentrales Thema im 21. Jahrhundert. Eine vielversprechende Technologie in diesem Zusammenhang ist die Umwandlung von CO₂ und Wasserstoff (H₂) zu Methan (CH₄) und Wasser (H₂O) durch methanogene Archaea, da dieser biologische Prozess, mit der Reaktionsgleichung CO₂ + 4 H₂ = CH₄ + 2 H₂O, durch seine hohe Umwandlungseffizienz gekennzeichnet ist.

Wie die Reaktionsstöchiometrie zeigt, wird bei der Bildung von Methan gleichzeitig auch Wasser gebildet. Eine ständige Abtrennung von Wasser ist daher während einer kontinuierlichen Kultivierung von hydrogenotrophen methanogenen Archaea notwendig, auch dann, wenn keine zusätzlichen Flüssigkeiten in den Bioprozess eingespeist werden. Wenn diese Abtrennung durch eine konstante Entfernung von Fermentationsbrühe erzielt wird, kommt es zu einem Verlust an Biomasse und damit zu einer Reduktion von katalytischer Aktivität sowie zu einem Verlust von Medienkomponenten, beispielsweise von Salzen, Spurenelementen und Vitaminen.

In der WO2006119052 wird ein Verfahren zur Herstellung von Wasserstoff beschrieben, wobei flüchtige organische Säuren sowie Aceton und Butanol mittels Pervaporation abgetrennt werden. In der US2009117631 wird ein kontinuierliches Verfahren zur Gewinnung von Ethanol aus Zuckerrohr oder Getreide beschrieben, wobei durch Pervaporation dem Prozess Wasser entzogen wird. Die WO2006137808 beschreibt ein anaerobes Verfahren zur kontinuierlichen Behandlung von belasteten Abwässern unter Verwendung von aktiviertem Schlamm, der thermophile Bakterien enthält und in der WO2012133386 wird ein Verfahren zur Abwasserbehandlung mittels eines Filtersystems geoffenbart.

Eine selektive Entfernung von Wasser bei gleichzeitiger Beibehaltung der Biomasse und Medienkomponenten ist daher kostengünstiger und führt gegebenenfalls auch zu verbesserter Prozessleistung durch eine Steigerung der Menge an katalytisch aktiver Biomasse. Diese selektive Abscheidung von Wasser kann beispielsweise durch Membran-basierende Trennverfahren erreicht werden.

Die Erfindung umfasst somit ein Verfahren zur Durchführung eines Bioprozesses zur Herstellung von Methan durch Kultivierung von methanogenen Archaea, wobei der Bioprozess kontinuierlich und ohne zusätzliche Einspeisung von Flüssigkeit durchgeführt wird, dadurch gekennzeichnet, dass Wasser, welches zusätzlich gebildet wird, mittels eines Membranmoduls selektiv abgeschieden wird, so dass das Reaktorvolumen im Wesentlichen konstant gehalten wird.

Im Sinne dieser Erfindung bedeutet selektive Abscheidung von Wasser, dass bei dieser Abscheidung im wesentlichen nur Wasser abgetrennt wird, während das Fermentationsmedium und die Biomasse im Bioreaktor zurückgehalten werden. Beispielsweise enthält das abgetrennte Wasser keine Zellen und/oder Medienbestandteile. Sofern dem Bioprozess Wasser zugeführt werden muss, so kann dieses abgeschiedene Wasser gegebenenfalls der Fermentationsflüssigkeit wieder zugesetzt werden, wodurch wiederum geringere Betriebskosten entstehen.

Ein weiterer Aspekt der Erfindung ist das oben beschriebene Verfahren, dadurch gekennzeichnet, dass der Bioprozess kontinuierlich durchgeführt wird.

Prinzipiell ist ein kontinuierliches Fermentationsverfahren einer Batch-Fermentation ökonomisch weit überlegen, aber seine Durchführung setzt voraus, dass die kultivierten bzw. gezüchteten Organismen definiert schnell und gleichmäßig wachsen und sich vermehren, und dass sie unempfindlich gegen die Rühr- und Scherkräfte sind, die bei einem kontinuierlichen Fermentationsverfahren für eine längere Dauer auftreten und dadurch die biologischen Katalysator schädigen könnten.

Ein weiterer Aspekt der Erfindung ist das oben beschriebene Verfahren, dadurch gekennzeichnet, dass Biomasse und/oder Medienkomponenten im Bioreaktor zurückgehalten werden.

Ein weiterer Aspekt der Erfindung ist das oben beschriebene Verfahren, dadurch gekennzeichnet, dass das Verfahren bei einer Verdünnungsrate von 0,005 bis 0,2 h⁻¹ durchgeführt wird.

Ein weiterer Aspekt der Erfindung ist das oben beschriebene Verfahren, dadurch gekennzeichnet, dass das Membranmodul außerhalb oder innerhalb des Bioreaktors vorgesehen ist.

Als mögliche Membranmodule können herkömmliche Membranen von beliebigen Herstellern, wie beispielsweise Novasep, GE Osmonics, Hydranautics, Dow Water & Process Solutions, Toray, Koch Membrane Systems, Membrana, Polypore, Liquicell usw. eingesetzt werden. Wichtig ist, dass diese Membranmodule temperatur- und/oder chemisch stabil sind. In einer bevorzugten Ausführungsform wird ein Pervaporationsmodul verwendet. Es können aber auch Membranen in einen besonders für diese Anwendung hergestellten Halter eingebaut werden. Der erfindungsgemäße Bioprozess wird unter anaeroben Bedingungen durchgeführt, wobei während des Bioprozesses zusätzliches Wasser gebildet wird. Erfindungsgemäß werden methanogene Archaea kultiviert. Die methanogenen Archaea sind bevorzugt ausgewählt aus der Gruppe bestehend aus *Methanobacteriales, Methanomicrobiales, Methanopyrales*, *Methanococcales Methanosarcinales* und *Methanocellales*.
Ein methanogener Mikroorganismus, der von Natur aus in der Lage ist, Methan zu produzieren, ist Voraussetzung für jede Ausführungsform der Erfindung. Erfindungsgemäß ist der methanogene Mikroorganismus ein methanogenes Archaebakterium, umfassend alle Mitglieder der methanogenen Archaea Domäne, wie beispielsweise *Methanobacterium alcaliphilum, Methanobacterium bryantii*, *Methanobacterium congolense, Methanobacterium defluvii*, *Methanobacterium espanolae, Methanobacterium formicicum*, *Methanobacterium ivanovii*, *Methanobacterium palustre*, *Methanobacterium thermaggregans*, *Methanobacterium uliginosum, Methanobrevibacter acididurans*, *Methanobrevibacter arbor iphilicus, Methanobrevibacter gottschalkii*, *Methanobrevibacter olleyae*, *Methanobrevibacter ruminantium*, *Methanobrevibacter smithii*, *Methanobrevibacter woesei*, *Methanobrevibacter wolinii*, *Methanothermobacter marburgensis, Methanothermobacter thermoautotrophicus*, *Methanobacterium thermoautotrophicus, Methanothermobacter thermoflexus*, *Methanothermobacter thermophilics, Methanothermobacter wolfeii*, *Methanothermus sociabilis, Methanocorpusculum bavaricum, Methanocorpusculum parvum*, *Methanoculleus chikuoensis, Methanoculleus submarinus, Methanogenium frigidum, Methanogenium liminatans, Methanogenium marinum, Methanosarcina acetivorans, Methanosarcina barken, Methanosarcina mazei, Methanosarcina thermophila, Methanomicrobium mobile, Methanocaldococcus jannaschii*, *Methanococcus aeolicus*, *Methanococcus maripaludis, Methanococcus vannielii*, *Methanococcus voltaei*, *Methanothermococcus thermolithotrophicus* und *Methanopyrus kandleri*. Erfindungsgemäß wird durch den Bioprozess Methan hergestellt. Ein weiterer Aspekt der Erfindung ist das oben beschriebene Verfahren, dadurch gekennzeichnet, dass das Membranmodul temperatur- und/oder chemisch beständig ist.

### Kurze Beschreibung der Figuren:

Figur 1 zeigt den experimentellen Aufbau der Bioprozessanlage.
Figur 2 zeigt das Reaktorvolumen, die Biomassekonzentration, die gesamte Biomasse und qCH4 während des ersten Experiments.
Figur 3 zeigt das Reaktorvolumen, die Biomassekonzentration, die gesamte Biomasse und qCH4 während des zweiten Experiments.
Figur 4 zeigt den transmembranen Fluss im zweiten Versuch.

### Beispiele:

Die folgenden Beispiele sind rein exemplarisch und sollen die hier offenbarte Erfindung besser illustrieren. Änderungen und Variationen der folgenden Beispiele im Rahmen der vorliegenden Patentansprüche können durchgeführt werden.

Um die Machbarkeit einer Pervaporation zur selektiven Abtrennung von Wasser bei biologischen Verfahren zu zeigen, wurde *M. marburgensis* in einem Bioprozessreaktor kontinuierliche kultiviert, wobei der Bioprozessreaktor mit einem Bypass-Membranmodul außerhalb des Reaktors ausgestattet wurde. Die Fermentationsbrühe wurde kontinuierlich durch den Bypass gepumpt und so das Reaktorvolumen durch die kontinuierliche Abtrennung von Wasser über das Membranmodul konstant gehalten. Während des Bioprozesses wurden die Biomassekonzentration, die spezifische und volumetrische Produktivität sowie die Qualität des Permeats überwacht, um den Einfluss der selektiven Abscheidung des Wassers auf die Kultur zu untersuchen.

### Material und Methoden

### Fermentations-Setup und Medienzusammensetzung

Alle Experimente wurden mit *Methanothermobacter marburgensis* (Stamm DSM 2133) in einem 10 L Laborreaktor durchgeführt (Biostat C +, Sartorius Stedim Biotech AG, Göttingen, Deutschland). *M. marburgensis* Kulturen wurden bei 4 °C und 0,5 bar Überdruck eines H₂/CO₂ Atmosphäre in druckfesten Flaschen, welche mit einem Kautschuk-Gummi Pfropfen verschlossen sind, gelagert. Die Gasphase wurde einmal pro Woche durch Ablassen des Überdrucks in der Flasche und Wiederbefüllen mit H₂/CO₂ auf einen Druck von 0,5 bar ausgetauscht.

Für die Fermentation wurde ein leicht verändertes Medium nach Schönheit (Schoenheit et al, 1979) verwendet. Folgende Bestandteile pro Liter sind enthaltend:
2,1 g NH₄Cl, 6,8 g KH₂PO₄, 3,6 g Na₂CO₃, 0,09 g Titriplex I, 0,04 g MgCl₂·6H₂O, 0,01 g FeCl₂·4H₂O, 0,2 mg CoCl₂·6H₂O, 1,2 mg NiCl₂·6H₂O, 0,2 mg NaMoO₄·2H₂O.

Dasselbe Medium wurde während der kontinuierlichen Kultivierung zugeführt, wobei zusätzlich 100 µl/L Antischaummittel beigefügt waren.

Um anaerobe Bedingungen im Reaktionsgefäß zu gewährleisten, wurde das gesamte System mit einer H₂/CO₂ Mischung für mehrere Minuten vor der Inokulation gespült. Zum Beimpfen wurden 50 ml der *M. marburgensis* Stammlösung pro Liter Medium anaerob in den Reaktor unter Verwendung einer anaeroben gasdichten Spritze übergeführt.

Die Kultivierung wurde bei 65 °C und einer Rührergeschwindigkeit von 1500 UpM durchgeführt. Der pH-Wert wurde durch eine pH-Sonde (Mettler Toledo GmbH, Wien, Österreich und Hamilton Bonaduz AG, Bonaduz, Schweiz) gemessen und durch Zugabe einer 1 M (NH₄)₂CO₃ Lösung bei einem Wert von 6,85 konstant gehalten, um die Ansäuerung durch das mikrobielle Wachstum zu kompensieren.

Das Oxidations-Reduktions-Potential (ORP) wurde durch eine Redox-Sonde (Mettler Toledo GmbH, Wien, Österreich) gemessen und befand sich stets unter-300 mV. Eine 0,5 M Na₂S 9H₂O Lösung wurde als Schwefelquelle verwendet und ständig in den Reaktor mit einer Rate von 0,018 LL-1 d-1 zugeführt. Die Zugabe von Lauge und Na₂S Lösung wurde gravimetrisch erfasst. Das Feedmedium wurde unter Verwendung einer analogen Peristaltikpumpe (Preciflow, Lambda Laboratory Instruments, Zürich, Schweiz) zugeführt. Die Durchflussrate wurde gravimetrisch erfasst und durch die Steuerung der Drehzahl konstant gehalten. Das Bioreaktorvolumen wurde durch Abtrennen der Kultursuspension über ein Tauchrohr unter Verwendung einer peristaltischen Pumpe konstant gehalten und durch das festgelegte Gewicht des Reaktors gesteuert. Die abgetrennte Suspension wurde in einer Ernteflasche gesammelt und deren Volumen gravimetrisch bestimmt. Alle Lösungen wurden durch Spülen mit N₂ oder H₂/CO₂ anaerob gehalten. Um die anaeroben Bedingungen aufrecht zu erhalten, wurden alle Flaschen mit 1 bar N₂ beaufschlagt. Reiner H₂ und CO₂ wurden als Substrate für *M*. *marburgensis* verwendet. Die Gasflüsse wurden individuell mittels eines thermischen Massendurchflussreglers (Brooks Instruments, Hatfield, USA) eingestellt.

### Pervaporation

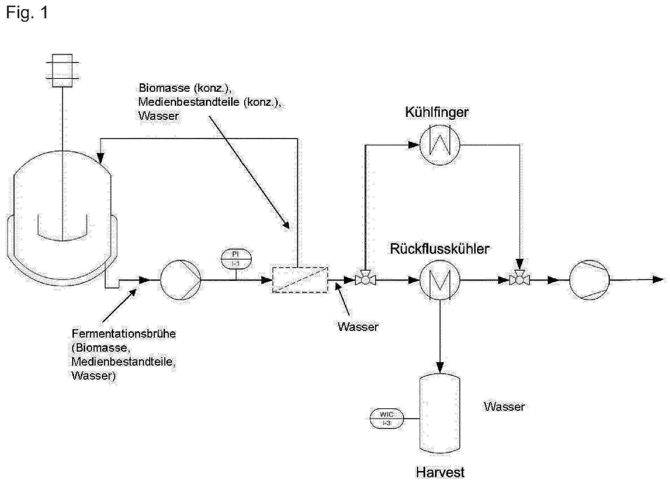

Für die selektive Abtrennung von Wasser wurde ein herkömmliches Pervaporationsmodul verwendet. Die Fermentationsbrühe wurde daher mit einer konstanten Durchflussrate von 20 L/h mittels eines Bypasses über das außerhalb des Reaktors befindliche Membranmodul mit einer Quattroflow 150S Pumpe (Quattroflow Fluid Systems GmbH Co KG, Hardegsen-Hevensen, Deutschland) gepumpt und das Retentat in den Reaktor zurückgeführt. Die anaeroben Bedingungen im Bypass wurden für die ganze Prozesszeit gewährleistet. Kontinuierliches Abtrennen von Wasser durch die Membran wurde durch Anlegen eines Vakuums von 100 mbar auf der Permeatseite des Membranmoduls erreicht. Das verdampfte Wasser wurde anschließend mit einem Lauda Alpha Kryostaten (LAUDA DR. R. WOBSER GMBH & CO KG, Lauda-Königshofen, Deutschland) bei ursprünglich -8 °C kondensiert. Der Aufbau ist in Figur 1 dargestellt.

### Ergebnisse und Diskussion

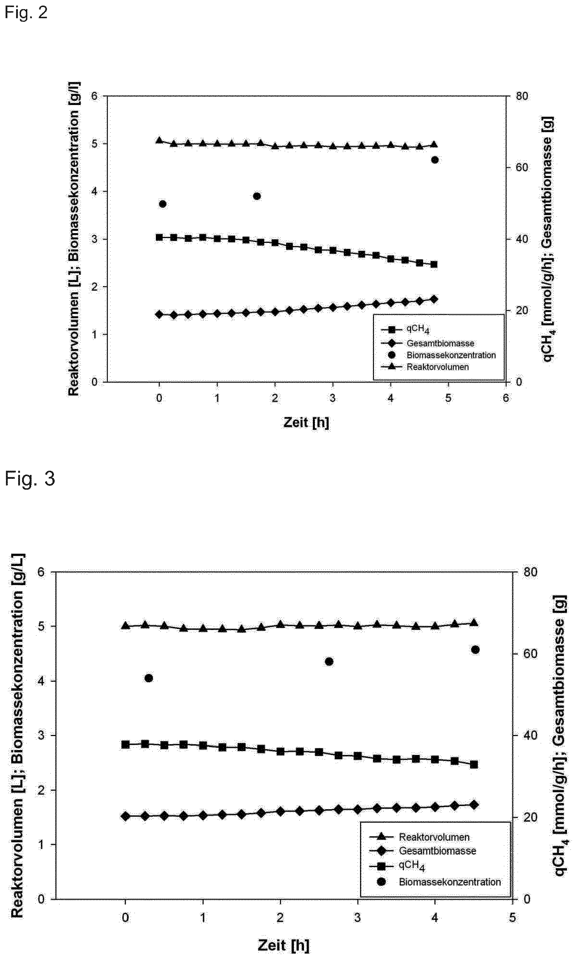

Figur 2 zeigt die Auswirkung der selektiven Abtrennung von Wasser über ein Pervaporationsmodul während der kontinuierlichen Kultivierung von *M. marburgensis*. Die gesamte Begasung betrug 0,5 vvm bei einem H₂:CO₂-Verhältnis von 4:1 im Reaktionsgas. Die Pervaporation wurde zum Zeitpunkt 0 gestartet.

Die Biomasse-Konzentration stieg während des Experiments von 3,73 auf 4,65 g/L. Gleichzeitig sank die spezifische Methanproduktionsrate (qCH₄) kontinuierlich, was auf eine gaslimitierte Kultur zurückzuführen ist. Das abgetrennte Permeat war eine klare, transparente, wässrige Flüssigkeit, die sich signifikant von dem Überstand der Reaktionsbrühe nach Mikrofiltration unterschied, welche eine intensive gelbe Farbe hatte. Es ist daher davon auszugehen, dass die Medienkomponenten effektiv zurückgehalten wurden.

Das Experiment wurde wiederholt. Das Ergebnis ist in Figur 3 dargestellt.

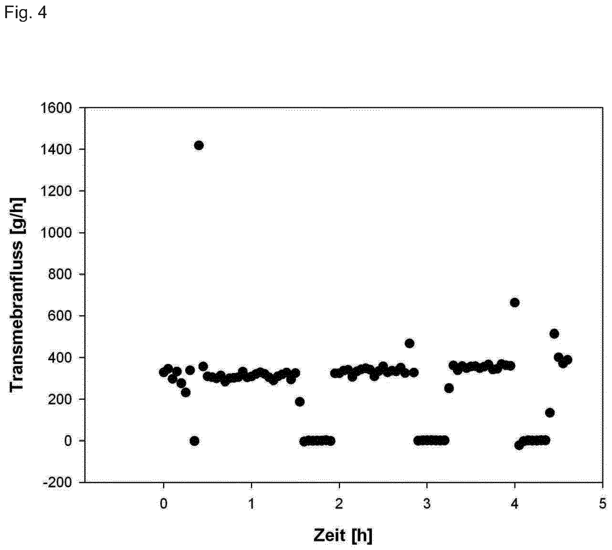

Bedauerlicherweise lieferte der Kryostat eine ungenügende Kühlleistung und es konnte keine stabile Temperatur gehalten werden. Die Temperatur stieg in den ersten eineinhalb Stunden von -8 auf 7 °C. Als Folge wurde das Pervaporationsmodul diskontinuierlich eingesetzt, um das Kryostat von Zeit zu Zeit abkühlen zu lassen (Figur 4). Während der Abschaltphasen wurde das Reaktorvolumen mittels eines normalen Tauchrohrs konstant gehalten, wobei entsprechende Mengen der Fermentationsbrühe abgetrennt wurden. Dies führte zu einem etwas geringeren Anstieg der Biomassekonzentration während des Experiments (4.05 g/L auf 4.57 g/L) im Vergleich zu vorstehendem Experiment.

Die erfolgreiche, selektive Abtrennung von Wasser aus kontinuierlichen Kulturen von *M. marburgensis* mit Pervaporation wurde gezeigt. Diese hatte keine negativen Auswirkungen auf die Kulturleistung. Die Biomassekonzentration wurde während der Experimente kontinuierlich gesteigert und der angepasste Transmembranfluss erlaubte ein konstantes Reaktorvolumen. Das Permeat war eine klare, wässrige Flüssigkeit, die sich in der Farbe deutlich von der Fermentationsbrühe unterschied, welche sonst auf der Permeatseite durch Mikrofiltration abgetrennt wurde. Weiter konnte auch ein effektives Zurückhalten der Medienkomponenten gezeigt werden.

## Patentansprüche

1. Verfahren zur Durchführung eines Bioprozesses zur Herstellung von Methan durch Kultivierung von methanogenen Archaea, wobei der Bioprozess kontinuierlich und ohne zusätzliche Einspeisung von Flüssigkeit durchgeführt wird, **dadurch gekennzeichnet, dass** Wasser, welches zusätzlich gebildet wird, mittels eines Membranmoduls durch Pervaporation selektiv abgeschieden wird, so dass das Reaktorvolumen im Wesentlichen konstant gehalten wird.

2. Verfahren nach Anspruch 1, **dadurch gekennzeichnet, dass** Biomasse und/oder Medienkomponenten im Bioreaktor zurückgehalten werden.

3. Verfahren nach einem der Ansprüche 1 oder 2, **dadurch gekennzeichnet, dass** das Verfahren bei einer Verdünnungsrate von 0,005 bis 0,2 h⁻¹durchgeführt wird.

4. Verfahren nach einem der Ansprüche 1 bis 3, **dadurch gekennzeichnet, dass** die methanogenen Archaea ausgewählt werden aus der Gruppe bestehend aus *Methanobacteriales, Methanomicrobiales, Methanopyrales*, *Methanococcales*, *Methanosarcinales* und *Methanocellales*.

## Claims

1. A method of conducting a bioprocess for the production of methane by cultivating methanogenic archaea, wherein the bioprocess is carried out continuously and without an additional supply of fluid, **characterized in that** water, which is formed in addition, is selectively separated by means of a membrane module through pervaporation, so that the reactor volume is kept substantially constant.

2. The method according to claim 1, **characterized in that** biomass and/or media components are retained in the bioreactor.

3. The method according to one of claims 1 or 2, **characterized in that** the method is carried out at an evaporation rate of 0.005 to 0.2 h⁻¹.

4. The method according to one of claims 1 to 3, **characterized in that** the methanogenic archaea are selected from the group comprising *methanobacteriales*, *methanomicrobiales*, *methanopyrales*, *methanococcales*, *methanosarcinales* and *methanocellales*.

## Revendications

1. Procédé permettant la mise en oeuvre d'un bio-processus destiné à produire du méthane par mise en culture d'archées méthanogènes, ledit bio-processus étant mis en oeuvre de manière continue et sans ajout de liquide, **caractérisé en ce que** l'eau qui se forme de manière supplémentaire est séparée par pervaporation sélective au moyen d'un module à membrane, permettant ainsi de maintenir un volume sensiblement constant dans le réacteur.

2. Procédé selon la revendication 1, **caractérisé en ce que** la biomasse et/ou les constituants du milieu sont retenus dans le bioréacteur.

3. Procédé selon l'une des revendications 1 ou 2, **caractérisé en ce que** ledit procédé est mis en oeuvre à un taux de dilution compris entre 0,005 et 0,2 h⁻¹.

4. Procédé selon l'une des revendications 1 à 3, **caractérisé en ce que** les archées méthanogènes sont choisies dans le groupe constitué de *Methanobacteriales*, *Methanomicrobiales*, *Methanopyrales*, *Methanococcales*, *Methanosarcinales* et *Methanocellales*.
